(19) Europäisches Patentamt / European Patent Office / Office européen des brevets

(11) **EP 2 655 642 B1**

(12) **EUROPEAN PATENT SPECIFICATION**

(45) Date of publication and mention
of the grant of the patent:
**01.02.2017 Bulletin 2017/05**

(51) Int Cl.:
*C12P 7/06* *(2006.01)*     *A01N 59/00* *(2006.01)*

(21) Application number: **11810745.7**

(22) Date of filing: **20.12.2011**

(86) International application number:
**PCT/US2011/066294**

(87) International publication number:
**WO 2012/088180 (28.06.2012 Gazette 2012/26)**

(54) **USE OF SYNERGISTIC FORMULATIONS CONTAINING STABILIZED CHLORINE DIOXIDE AND PEROXIDE TO REDUCE GROWTH OF CONTAMINANT MICROORGANISMS IN ETHANOL FERMENTATION**

VERWENDUNG SYNERGISTISCHER FORMULIERUNGEN ENTHALTEND STABILISIERTES CHLORDIOXID UND PEROXID ZUR VERMINDERUNG DES WACHSTUMS VERUNREINIGENDER MICROORGANISMEN IN DER ETHANOL-GÄRUNG

L'UTILISATION DE FORMULATIONS SYNERGIQUES COMPRENANT DU DIOXYDE DE CHLORE STABILISÉ ET DU PEROXYDE POUR RÉDUIRE LA CROISSANCE DE MICRO-ORGANISMES CONTAMINANTS DANS LA FERMENTATION ALCOOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2010 US 201061425032 P**

(43) Date of publication of application:
**30.10.2013 Bulletin 2013/44**

(73) Proprietor: **E. I. du Pont de Nemours and Company**
**Wilmington, DE 19805 (US)**

(72) Inventors:
• **SOLOMON, Ethan, Baruch**
**Wilmington, Delaware 19810 (US)**
• **OKULL, Derrick, Otieno**
**Pleasanton, California 94588 (US)**

(74) Representative: **Towler, Philip Dean**
**Dehns**
**St Bride's House**
**10 Salisbury Square**
**London EC4Y 8JD (GB)**

(56) References cited:
**WO-A2-2007/149450      CA-A1- 2 300 807**
**US-A1- 2002 064 565      US-A1- 2010 291 649**

• **KRAM, J.W.: "PureMash system provides antibiotic free antimicrobial solution", Ethanol Producer Magazine, April 2008 (2008-04), XP002674231, Retrieved from the Internet: URL:http://www.ethanolproducer.com/article s/4028/puremash-system-provides-antibiotic -free-antimicrobial-solution**

**Description**

**FIELD OF THE INVENTION**

The present invention relates to synergistic combination of stabilized chlorine dioxide and peroxide compound and method of use, particularly in fermentation processes.

**BACKGROUND OF THE INVENTION**

In the last decade, the use of ethanol as a transportation fuel has increased significantly. Ethanol production in the United States rose from approximately 6.4 billion liters in the year 2000 to over 37 billion liters in 2009. The number of ethanol plants increased from 54 in 2000 to 170 in 2009. Similar increases in production and plant construction have occurred in Latin America and Europe. In 2007, the United States Congress enacted the Energy Independence and Security Act (H.R. 6), which set the renewable fuel standard at 136 billion liters of ethanol by the year 2022. If this standard is to be met, the ethanol industry will continue to grow.

Currently both industrial ethanol (e.g., fuel) and beverage ethanol are produced on large scale from agricultural (natural) feedstocks by fermentation processes in which sugar is converted to ethanol and carbon dioxide by inoculant yeast. Many feedstocks can be used to provide the sugar for fermenting, including potentially, any starch or cellulosic material, which includes nearly all plants, as any starch or cellulose can be a precursor to sugar. Some of the common feedstocks particularly suitable for producing fuel ethanol include corn, milo, sorghum, sugar cane, sugar beets and molasses.

The feedstocks used for ethanol production are natural products therefore, a wide variety of microorganisms such as bacteria, fungi, and yeasts are likely to be naturally present in the feedstocks. Commercial fermentation process conditions are not completely sterile, hence these "contaminant microorganisms" will be present in the process. In commercial ethanol production, microorganisms of greatest concern are lactic acid-producing bacteria and acetic acid-producing bacteria. Such bacteria enter the process from several sources including raw materials, equipment, process water, air, and inoculant yeast, among others. Concentrations of such bacteria may increase in the process environment either through introduction with incoming materials (raw materials, water, air, yeast) or naturally proliferate as a result of conditions favorable to bacterial growth. The optimum atmosphere for yeast production is also extremely conducive to the growth of these bacteria. Organic acids produced by the bacteria inhibit the growth of yeasts and thus reduce ethanol production rate. The bacteria may also consume sugars and other nutrients intended for use by the yeast to produce desired products, rendering the entire process less economical.

Many fermentation processes use antibiotics as antimicrobial compositions. Such use has become disfavored due to suspected development of antibiotic-resistant bacteria and accumulation of antibiotic residues in fermentation byproducts. Antibiotic-resistant bacteria are a significant concern in human health.

Byproducts of ethanol production include solids that are collected after distillation of the ethanol product. Such solids include distillers dried grains with solubles (DDGS) and distiller's wet grains with solubles (DWGS). Many countries are considering regulatory actions that would limit or eliminate the use of antibiotics for ethanol production. DDGS and DWGS are sold as animal feed products.

The need for antimicrobial treatments is increasing, not only because of the growth in production volume of ethanol but also the expansion in size of ethanol production facilities. Whereas a plant producing 150-200 million liters per year (MMly) was considered a large facility just a few years ago, 380 MMly (or more) facilities are today's industry standard. In fed-batch processes, the volume of individual fermentation batches has increased significantly. To accommodate this added capacity, the flow rate of feedstock (commonly known as "mash" once it has been prepared for entry into fermentation) into a fermentation system has increased from approximately 2000-3000 liters per minute to 4500-6000 liters per minute in the largest ethanol production facilities.

WO 2007/149450 describes the use of stabilized chlorine dioxide (SCD) to prevent bacterial contamination in ethanol production. SCD is added prior to the onset of significant bacterial growth in ethanol production, as a preventive rather than as a remedial measure. The growth of contaminant bacteria prior to and during the fermentation of sugar to alcohol is thus substantially prevented, creating conditions that enhance growth of inoculant yeast and enable inoculant yeast to produce ethanol without inhibition by organic acids produced by the bacteria.

WO 2007/149450 and US 2010/291649 disclose a method for controlling growth of contaminant microorganisms in a fermentation process comprising adding the component: (a) a stabilized chlorine dioxide to one or more steps of a fermentation process, wherein the fermentation process comprises: (i) providing a fermentation vessel, (ii) providing a yeast inoculant, a fermentable sugar and process water, (iii) introducing separately, or in any combination, the inoculant, fermentable sugar and process water into a fermentation vessel to provide a fermentation broth, and (iv) contacting the inoculant with the fermentable sugar in the fermentation vessel at a temperature at which the inoculant converts the fermentable sugar to ethanol.

CA 2 300 807 discloses a method for controlling growth of contaminant microorganisms in a fermentation process comprising adding the component: (b) a peroxide compound to one or more steps of a fermentation process, wherein the fermentation process comprises: (i) providing a fermentation vessel, (ii) providing a yeast inoculant, a fermentable sugar and process water, (iii) introducing separately, or in any combination, the inoculant, fermentable sugar and process water into a fermentation vessel to provide a fermentation broth, and (iv) contacting the inoculant with the fermentable sugar in the fermentation vessel at a temperature at which the inoculant converts the fermentable sugar to ethanol.

US 2002/064565 discloses formulations comprising (a) a stabilized chlorine dioxide and (b) a peroxide compound and their use to provide an antibacterial activity for the treatment of medical conditions.

Although some methods are known, there remains demand for improved methods for addressing contaminant microorganisms in the fermentation industry, both for carbohydrate-containing feedstocks and in fermentation processes. An improved method should preferably be antibiotic-free and not result in residues that accumulate in fermentation coproducts or give rise to antibiotic-resistant bacteria. The method should be efficacious at a wide variety of pH ranges and conditions encountered in the fermentation industry. The method should also use treatment that has a reasonable shelf life, prior to use. There is also a need to improve economics of today's larger volume fermentation processes. The present invention meets these needs.

## SUMMARY OF THE INVENTION

The present invention provides a method for controlling growth of contaminant microorganisms in a fermentation process using a combination of (a) stabilized chlorine dioxide (SCD) and (b) a peroxide compound (PC). The method comprises adding SCD and PC, such as hydrogen peroxide, to one or more steps of a fermentation process, wherein the fermentation process comprises (i) providing a yeast inoculant, a fermentable sugar and process water; (ii) introducing separately, or in any combination, the inoculant, fermentable sugar and process water into a fermentation vessel to provide a fermentation broth; (iii) contacting the inoculant with the fermentable sugar in the fermentation vessel at a temperature at which the inoculant converts the fermentable sugar to ethanol. Optionally nutrients are added to one or more of the yeast inoculant, fermentable sugar and process water. Nutrients may also be added directly to the fermentation vessel. The SCD and PC may be added to the yeast inoculant, fermentable sugar or process water prior to introducing each of these to the fermentation vessel. Alternatively the SCD and PC may be added directly to the fermentation vessel.

The stabilized chlorine dioxide and peroxide compound, may be added separately to the process. When adding the components separately, the components may be added simultaneously or sequentially in any order. That is, components may be added at the same time (simultaneously) or one component may be added prior to adding the other component (sequentially). Alternatively, the method may comprise contacting the components (a) and (b) prior to adding to the fermentation process.

The present invention provides a method for controlling the growth of contaminant microorganisms in the reactants (defined hereinbelow) in the fermentation broth, and in the products of a fermentation process. The method also controls growth of contaminant microorganisms on surfaces of components of a fermentation system. The method consists of, consists essentially of, or comprises the step of adding stabilized chlorine dioxide and peroxide compound to a reactant or the fermentation broth, or to a surface or into a vessel of the fermentation system.

The present disclosure also provides a combination of components that can be used in cleaning-in-place (CIP) applications to treat surfaces of equipment used in fermentation processes. By "CIP" is meant herein that surfaces can be cleaned without the need to disassemble the equipment.

The method includes adding a combination of components which comprise SCD and PC in amounts effective to control the growth of contaminant microorganisms without detrimental effect on the yeast inoculant used in the fermentation process. The effective amount varies in accordance with how the combination of components is used, but can be determined by one skilled in the art in view of the disclosures herein. SCD is typically added in an amount to provide a concentration of SCD in the fermentation broth of 0.0002% to 5%, by weight, based on the total volume of the fermentation broth. PC is also typically added in an amount to provide a concentration of PC in the fermentation broth of 0.0002% to 5%, by weight, based on the total volume of the fermentation broth.

The method of the present invention further provides a method to control growth of at least one contaminant microorganism in a carbohydrate feedstock wherein the step of providing a fermentable sugar comprises providing a carbohydrate feedstock and contacting the feedstock with SCD and PC, wherein the carbohydrate content of the feedstock is at least 1% and preferably 1 to 70%, by weight, based on the total feedstock weight and wherein SCD and PC are added in effective amounts. Typically SCD is added in an amount of 10 ppm to 1000 ppm of SCD, based on total weight of the feedstock, and PC is typically added in an amount of 10 ppm to 1000 ppm of the peroxide compound, based on the total weight of the feedstock.

**DETAILED DESCRIPTION OF THE INVENTION**

The present invention provides methods for controlling the growth of a contaminant microorganism comprising, consisting essentially of, or consisting of, adding a combination of components comprising stabilized chlorine dioxide (SCD) and peroxide compound (PC) to one or more steps of a fermentation process. Synergistic combinations of SCD and PC are added to the process in amounts effective to control growth of contaminant microorganisms without inhibiting the ability of inoculant yeast to convert fermentable sugars into ethanol and carbon dioxide. SCD and PC are typically added to provide a concentration of SCD in the fermentation broth of 0.0002 to 5% by weight, and a concentration of PC in the fermentation broth of 0.0002 to 5% by weight, each based on the total volume of a fermentation broth.

The present invention further provides in the step of providing a fermentable sugar, a method to control growth of at least one contaminant microorganism in a carbohydrate feedstock comprising, consisting essentially of, or consisting of, providing a carbohydrate feedstock and adding stabilized chlorine dioxide and peroxide compound to the feedstock.

Definitions

The following terms have the definitions as provided below for use herein.

Aqueous medium means the medium is substantially water, such as for example greater than 80% water, preferably greater than 90% water, more preferably greater than 95% water. The aqueous medium can be greater than 99% water. Process water is an example of an aqueous medium.

Carbohydrate feedstock means a feed used in preparation of or directly as a fermentable sugar. That is, a carbohydrate feedstock is a fermentable sugar or a composition comprising a fermentable sugar or a composition that can be converted to a fermentable sugar.

The carbohydrate feedstock may comprise up to 100% by weight of carbohydrates. Generally the carbohydrate feedstock comprises between 1% and 70% carbohydrate based on the total weight of the feedstock, preferably between 2 and 40%, as a solution or suspension in an aqueous medium. The amount and composition of the carbohydrates in the feedstock can vary depending on the intended end use. For example, corn steep liquor, which is a carbohydrate solution obtained from a wet mill process, may comprise 16.5% carbohydrates. In a wet mill process, corn is soaked or steeped and then separated into various components. The corn steep liquor is the aqueous liquid obtained after the corn has been soaked for an extended period, during which readily fermentable soluble components are extracted from the corn solids into the steep water. The starch component from the wet mill process may comprise up to 40% by weight carbohydrates.

The carbohydrate feedstock may comprise other components generally functioning as adjuncts to the solutions and/or suspensions. For example, the carbohydrate feedstock may comprise enzymes, surfactants, dispersants, antifoaming compositions, minerals, trace elements, and combinations of two or more thereof. These components and other components that act as adjuncts are well-known to those skilled in the art.

Control as applied to growth of a microorganism herein means to reduce and/or prevent the growth of a targeted undesirable contaminant microorganism. Controlling the growth of a microorganism as used herein also includes to maintain the microorganism population at a desired level, to reduce the population of the microorganism to a desired level (partially reduce or even reduce to undetectable limits, e.g., zero population), and/or to inhibit growth of the microorganism.

An effective amount refers herein to an amount of each of the SCD and PC that, in combination, when added to a fermentation process, is effective to control the growth of contaminant microorganisms without detrimental effect on the yeast inoculant used in the fermentation process.

A fermentable sugar is a reactant and common nutrient for yeast inoculants used in ethanol fermentation processes. The fermentable sugar is a sugar (e.g. glucose) or starch that is converted by action of yeast to ethanol and carbon dioxide. Equation (1) illustrates the process for glucose ($C_6H_{12}O_6$).

$$C_6H_{12}O_6 \rightarrow 2\ C_2H_5OH + 2\ CO_2 \qquad (1)$$

A fermentable sugar as used herein is a carbohydrate that is derived from essentially any plant source comprising sugar, starch and/or cellulose, generally provided in the form of a solution or suspension of the sugar, starch and/or cellulose in water. Starch and/or cellulose can be converted by processes known in the art, e.g., using enzymes, to a fermentable sugar for use in the method of this invention. The fermentable sugar can be derived from sources of starch or one or more cellulosic material such as corn, sorghum, wheat, wood chips, milo, barley, millet, sugar cane, sugar beets, molasses, whey, potatoes, wheat straw, corn stover, switch grass, algae, seaweed, and/or other biological sources. The fermentable sugar may also be derived from fruit juice (e.g., grapes, apples). The fermentable sugar may alternatively be derived from non-traditional feedstocks such as wood waste, bagasse, paper waste, and municipal solid waste. Processes are known to those skilled in the art to convert these sources to fermentable sugar. For reference, see, J. Y.

Zhu, et al. Bioresource Technology (2010) vol. 101 (13), pp. 4992-5002; and A. L. Demain, J. Ind. Microbiol. Biotechnol. (2009) vol. 36(3), pp. 319-332.

Conveniently, the fermentable sugar is derived from corn, using either the dry grind or wet mill process. In a dry grind process, corn is ground into meal and processed without separation into its constituent components comprising essentially fiber, starch, oil, and protein. In a wet mill process, corn is soaked or steeped in water and then mechanically separated into its constituent components. The corn starch component from the wet mill process or meal (corn flour) from the dry grind process is mixed with water and enzymes and cooked to solubilize the starch.

Corn starch is a polysaccharide, that is, a polymer made of individual units of glucose. The corn starch is converted to smaller (shorter) polysaccharides, i.e., dextrins, by enzymes ($\alpha$-amylase). The smaller polysaccharides are converted to glucose (monosaccharide) using the enzyme glucoamylase, thus forming the fermentable sugar.

As an alternative to corn, the fermentable sugar can be derived from molasses. Molasses can be obtained from a variety of sources including sugar cane or sugar beets, for example, as a byproduct of the process to manufacture crystalline sugar. Molasses is typically obtained as a syrup, to which other ingredients may be added in preparation for fermentation. These other ingredients include sugarcane juice, beet juice, water, and vitamins or other nutrients. Whether one or more of the other ingredients are added and the amount added will vary in a molasses-derived fermentable sugar.

As an alternative to corn, the fermentable sugar can be derived from sugar cane juice. Sugar cane juice is juice extracted with water from sugar cane. The extraction of juice from sugarcane is also accomplished by physical crushing, diffusion in water, or other methods generally well known to those skilled in the art. See, for example, H. V. Amorim, et al, in "The Alcohol Textbook" (2009), Nottingham University Press , Nottingham, pp. 39-46; and EPA Food and Agricultural Industries Handbook, chapters 9.10.1.1 (sugar cane) and 0.10.1.2 (sugar beets), available at http://www.epa.gov/ttnchie1; ap42/ch09/ (accessed December 18, 2011). Sugar cane juice can be used without further processing and added directly to a fermentation vessel as the fermentable sugar.

For purposes herein, steps used to produce carbohydrate feedstocks and fermentable sugars are steps of the fermentation process. That is, the fermentation process comprises steps to produce carbohydrate feedstocks, and steps to convert starch and/or cellulose to a fermentable sugar.

In the fermentation process, carbohydrates, including sugars and starches, is typically present in the fermentation broth at a concentration of about 5 to about 40% (weight/volume), preferably in the range of about 10 to 35% (weight/volume), based on the total volume of the fermentation broth.

Fermentation broth as used herein means the contents of a fermentation vessel during the fermentation process after all reactants have been added and typically comprises fermentable sugar, yeast inoculant, optional nutrients and process water.

Fermentation process as used herein means a process comprising contracting a yeast inoculant, with a fermentable sugar, and optional nutrients, in process water to produce ethanol. A fermentation process can be batch or continuous. In addition, for purposes herein, fermentation also includes steps for preparing or pretreating one or more of the reactants, such as processes to prepare the fermentable sugar from natural sources, and yeast propagation.

The contacting step is performed at a temperature at which the yeast inoculant converts sugar to ethanol and carbon dioxide.

When ethanol is produced via a dry grind process using corn as a feedstock, the fermentation process may comprise one or more of the steps of slurrying of the corn, liquefaction, saccharification, preparing inoculant yeast (propagation), fermenting the mash(actual step of action of inoculant on sugar to produce ethanol), separating and recovering the resultant ethanol, and optionally recycling the spent inoculant yeast. The fermentation process may comprise one or more of the steps of preparing inoculant yeast, preparing the juice by dilution, pasteurization, or other such process, fermenting, separating and recovering the ethanol, and optionally recycling the yeast. It will be understood by those skilled in the art that these process steps may vary, depending on feedstock availability and plant design and plant location.

Fermentation system as used herein comprises components (equipment), such as vessels and pipes in which and through which one or more of the reactants and products of a fermentation process resides or passes. Such equipment have surfaces on which bacteria and other contaminant (undesirable) microorganisms may be present. As part of a fermentation system is a fermentation vessel - the vessel in which the fermentation step of reacting a fermentable sugar with yeast inoculant to produce ethanol occurs.

When ethanol is produced via a dry grind process, the fermentation system may comprise one or more vessels such as a slurry tank, liquefaction tank, saccharification tank, yeast propagation tank, and fermentation vessel. Such vessels and their use are known to those skilled in the art. See, for example, R. J. Bothast and M. A. Schlicher, Applied Microbiology and Biotechnology (2005), vol. 67(1), pp. 19-25. When ethanol is produced via a wet mill process, the fermentation system may comprise one or more vessels such as a steep tank, a separation tank, yeast propagation tank, and fermentation vessel. It will be understood by those skilled in the art that these process steps may vary, depending on feedstock availability and plant design and plant location.

A yeast inoculant is any microorganism added purposely to a process in order to convert a feedstock (input) to a desired product (output). For purposes herein, a yeast inoculant is a microorganism which is capable of converting a fermentable sugar to ethanol. Yeasts are common inoculants used in ethanol fermentation. Yeasts are microorganisms capable of living and growing in either aerobic (with oxygen) or anaerobic (lacking oxygen) environments. An inoculant may also be selected from the group consisting of fungi, and algae that are capable of converting a fermentable sugar to ethanol.

The following discussion is directed to a process in which the inoculant is yeast.

For purposes herein, an inoculant yeast is a yeast which has been deliberately selected for a particular conversion in a reaction system. For example, an inoculant yeast may be selected for production of additional yeast in yeast propagation (such as for use as baker's yeast); an inoculant yeast may be selected for metabolism of a particular nutrient for production of enzymes. In this specific application, an inoculant yeast is selected for fermentation of a fermentable sugar to produce ethanol of desired quality and in desired quantity. Inoculant yeasts are generally selected because of their ability to completely ferment available sugars, tolerance to high levels of osmotic stress, elevated temperatures, and high concentrations of ethanol. Yeast are commonly used in ethanol fermentation. Yeast are microorganisms capable of living and growing in either aerobic (with oxygen) or anaerobic (lacking oxygen) environments. Suitable inoculant yeasts for use in the process of this invention include, but are not limited to *Saccharomyces cerevisiae* (*S. cerevisiae*), *Saccharomyces uvarum* (*S. uvarum*), *Schizosaccharomyces pombe* (*S. pombe*), and *Kluyveromyces sp.*

The inoculant yeast may be introduced into the fermentation vessel as a yeast inoculum, which is an activated form of the inoculant yeast. That is, prior to introducing inoculant yeast into a fermentation vessel, a yeast inoculum is produced by contacting a yeast starter culture and a nutrient composition in a propagation tank to propagate (increase the quantity of) the yeast. The nutrient composition comprises one or more fermentable sugar, enzyme, additional nutrients, and water to grow or activate the yeast. The propagation tank is a separate vessel from the fermentation vessel and is operated at suitable conditions of temperature (e.g., 68-104°F, 20-40°C). Each inoculant will have preferred temperature for propagation. For example, a temperature of 30-32°C (86-90°F) may be particularly suitable for propagating *S. cerevisiae.* While it is recognized that yeast propagation can occur in the fermentation vessel during the fermentation process, activation of yeast in a propagation tank provides a highly active inoculant yeast. Thus, highly active yeast is introduced to the fermentation vessel. Such yeast propagation techniques are known to those skilled in the art. See, for example, E. Bellissimi, et al., Process Biochemistry (2005), vol. 40(6), pp. 2205-2213; and M. Knauf, et al., Sugar Industry (2006), vol. 131, pp. 753-758.

For continuous fermentation processes, there is often no separate yeast propagation tank. Yeast may or may not be recycled in a continuous process. When no recycle is available, yeasts grow and produce ethanol continuously in a stage called a primary fermentation. When recycle is available, such as is common when molasses or sugarcane juice is used as a feedstock for the fermentable sugar, yeasts are recycled by separation from the other fermentation components, (usually using centrifugation) and typically treated with acid in a separate tank (generally called a yeast recycle tank) to condition the yeast cells for a new fermentation cycle. Alternatively, the yeast may be separated from the fermentation broth, then dried and sold as a co-product. These steps are well known to those skilled in the art. See, for example, E. A. N. Fernandes, et al., Journal of Radioanalytical and Nuclear Chemistry (1998) vol. 234 (1-2), pp. 113-119; and L. C. Basso, et al., FEMS Yeast Res. (2008) vol. , pp. 1155-1163.

Relative to bacteria, yeasts may have moderate to slow fermentation rates. To compensate for their metabolic rate, large amounts of yeast may be required in large scale industrial ethanol production. Inoculant yeast is generally added to the fermentation process in an amount typically about $1 \times 10^5$ to $1 \times 10^7$ cells per gram of fermentation broth. It will be recognized by those skilled in the art that this amount may vary depending on the method of fermentation employed.

Mash is used herein to refer to a composition comprising a fermentable sugar. Mash includes any mixture of mixed grain or other fermentable carbohydrates in water used in the production of ethanol at any stage from mixing of a fermentable sugar with water to prior to any cooking and saccharification through to completion of fermentation, as defined in Jacques, K.A., Lyons, T.P., Kelsall, D.R, "The Alcohol Textbook", 2003, 423-424, Nottingham University Press, UK.

Microorganisms in the context of this invention are in two categories, desirable and contaminant (undesirable) microorganisms. A desirable microorganism has the capability of consuming nutrients to convert a fermentable sugar to ethanol. Desirable microorganisms include yeast inoculants such as the yeast, *Saccharomyces cerevisiae,* which is used in the fermentation of glucose into ethanol and carbon dioxide. Other desirable microorganisms are used in other biorefinery processes. Desirable microorganisms are not typically present in carbohydrate feedstocks. When desirable microorganisms are present in a carbohydrate feedstock, the number of viable cells present is typically too low to compete favorably with the other microorganisms commonly also present in the feedstock.

A contaminant microorganism is a microorganism that competes with the yeast inoculant for and consumes the fermentable sugar and nutrients. Contaminant microorganisms produce undesirable products and/or produce alcohol at a much lower rate than would be produced by the yeast inoculant.

Contaminant microorganisms include bacteria, fungi, wild or contaminant yeasts, and other microorganisms capable of metabolizing components of a carbohydrate feedstock to sustain the viability of the microorganism. Contaminant microorganisms contaminate carbohydrate feedstocks, consume the feedstock as a food source in support of their growth, multiply, and thus deplete the feedstock.

Contaminant microorganisms such as contaminant yeasts are often found in both industrial and beverage ethanol production, and can cause severe episodes of contamination, resulting in reduced ethanol productivity in a fermentation process. These unwanted microorganisms may be introduced into the process through the fermentable sugar (feedstock), process water, air, operators, and numerous other sources.

Contaminant microorganisms, such as bacteria, including lactic acid bacteria (species of *Lactobacillus*) produce products such as acetic and lactic acids from glucose feedstocks, that not only consume the feedstock and thus prevent feedstock conversion to desired products, but also adversely affect desirable microorganisms in a biorefining process. For example, acetic and lactic acids adversely affect the rate at which *Saccharomyces cerevisiae* converts glucose to ethanol. Other contaminant bacteria include species of *Pediococcus, Enterococcus, Acetobacter, Gluconobacter,* and *Clostridium.*

A <u>nutrient</u> means an element which supports growth and/or survival of the inoculant yeast for example a nutrient may be a source of carbon, nitrogen, oxygen, sulfur, phosphorus, magnesium, and a variety of trace elements such as metals. A carbon source can be an alcohol such as methanol, or a hydrocarbon such as octane. A carbon source may also be the fermentable sugar. A nitrogen source can be urea, ammonia, an ammonium salt or a nitrate. A salt such as magnesium sulfate can provide magnesium and sulfur. Phosphorus can be supplied as a sodium or potassium salt, while oxygen can be supplied by introducing air into the process. Other forms of gaseous oxygen can be used, such as pure oxygen or oxygen/nitrogen mixtures. Other elements can be added directly or may be naturally present in process components.

<u>Peroxide compounds,</u> as used herein, refers to compounds that contain at least one oxygen-oxygen single bond. Preferred peroxides are selected from the group consisting of hydroperoxides, organic peroxides, inorganic peroxides and peroxygen-releasing compounds.

Hydroperoxides are compounds of the general formula R-O-O-H wherein R is a hydrogen or straight, branched and/or cyclic alkyl radical having 1 to 20 carbons atoms and can be optionally interrupted by one or more oxygen and/or carbonyl groups. Examples of hydroperoxides include, but are not limited to, hydrogen peroxide, acetic peroxide, tert-butyl hydroperoxide, cumene hydroperoxide, and diethyl ether hydroperoxide.

Organic peroxides are compounds that have the general formula $R^1$-O-O-$R^2$, where $R^1$ and $R^2$ are independently straight, branched, and/or cyclic alkyl radical having 1 to 20 carbons atoms and can be optionally interrupted by one or more oxygen and/or carbonyl groups. Examples of organic peroxides include, but are not limited to, benzoyl peroxide and di-tert-butyl peroxide.

Inorganic peroxides are compounds that contain an $O_2^{2-}$ anion and have the general formula of $M_2O_2$ or $M^1O_2$ wherein M is an alkaline metal and $M^1$ is an alkaline earth metals. Examples of inorganic peroxides include, but not limited to, sodium peroxide, calcium peroxide, and barium peroxide.

Peroxygen-releasing compounds are also useful in the present invention. More particularly a nitrogen-free peroxygen-releasing compound is defined herein as any nitrogen-free material capable of releasing hydrogen peroxide or other oxygen-containing radicals upon addition to an aqueous system. Examples of peroxygen-releasing compounds include alkali metal, alkaline earth metal and transition metal compounds of percarbonates, perborates and peroxides, and suitable mixtures thereof. Preferred peroxygen-releasing compounds include, but are not limited to, sodium percarbonate, sodium perborate, sodium peroxide, calcium peroxide, magnesium peroxide and zinc peroxide.

Preferably, the peroxide used in the present invention is hydrogen peroxide, herein referred to as "HP". HP is commercially available in concentrations of 33 to 37 % by weight in water. Commercially available HP may also contain additives. Additives include stabilizers, such as phosphoric or other mineral acids, and inhibitors, such as pyrophosphate salts and stannate compounds.

<u>Preserve and preservation</u> means treating a carbohydrate feedstock to prevent reaction or consumption of carbohydrate by contaminant microorganisms such as bacteria. Preservation provides a stable carbohydrate feedstock that does not undergo substantial change, such as would result from reaction or consumption of the carbohydrates due to microbial metabolism, over a period of time of at least one month. One measure of change is the microbial population of the preserved feedstock. When properly preserved, the carbohydrate feedstock does not undergo an increase in the microbial population in the feedstock of more than 1 $\log_{10}$ CFU/ml or 1 $\log_{10}$ CFU/g. Typically microbial population is expressed as $\log_{10}$ CFU/ml for liquid feedstocks and as $\log_{10}$ CFU/g for solid/semi-solid feedstocks. The expression $\log_{10}$ CFU/g can also be used for liquid feedstocks.

A second measure of change with respect to preservation is based on the concentrations of particular acids in the feedstock. Acids are known products resulting from reaction and/or consumption of carbohydrates by contaminant bacteria. For example, certain bacteria known to contaminate carbohydrate feedstocks produce lactic acid and acetic acid upon consumption of the carbohydrate. Increasing concentrations of lactic acid and acetic acid in a carbohydrate feedstock can be used to indicate whether the feedstock is properly preserved. When properly preserved, concentration of lactic acid in the carbohydrate feedstock is less than 0.60% (weight/volume) and concentration of acetic acid in the carbohydrate feedstock is less than 0.30 % (weight/volume).

It will be further appreciated by those skilled in the art that other measures of change may be used. For example, detection of the presence of undesired compounds may indicate change, such as a product from metabolism of the carbohydrate feedstock. Detection methods may include spectrophotometry, chromatography, and other methods known to those skilled in the art. Still other measures may include physical changes to the carbohydrate feedstock such as specific gravity or viscosity.

Reactant means yeast inoculant, fermentable sugar, optional nutrients as well as any other optional components in a fermentation broth. For purposes herein, reactant also includes process water.

Stabilized chlorine dioxide otherwise referred to herein as SCD means one or more chlorine dioxide-containing oxy-chlorine complexes, one or more chlorite-containing compounds, one or more other entities capable of forming chlorine dioxide when exposed to acid, and combinations thereof. Thus, stabilized chlorine dioxide comprises at least one of a chlorine dioxide-containing oxy-chlorine complex, a chlorite-containing compound, or an entity capable of forming chlorine dioxide in a liquid medium when exposed to acid. SCD are defined herein are commercially available products.

A preferred chlorine dioxide-containing oxy-chlorine complex is selected from the group consisting of a complex of chlorine dioxide with carbonate, a complex of chlorine dioxide with bicarbonate and mixtures thereof. Examples of chlorite-containing compounds include metal chlorites, and in particular alkali metal and alkaline earth metal chlorites. A specific example of a chlorite-containing compound that is useful as SCD (a chlorine dioxide precursor) is sodium chlorite, which can be used as technical grade sodium chlorite.

SCD is preferably used in the form of an aqueous solution, such as that of an alkali metal or alkaline earth metal chlorite, typically sodium chlorite ($NaClO_2$). Sodium chlorite in solution is generally stable at pH above 7, but releases the active chlorine dioxide ($ClO_2$), when the pH is lowered below neutral (pH 7). The rate of activation of SCD, that is, the rate at which the active $ClO_2$ is released from the stable form, increases as pH decreases.

The exact chemical composition of many of SCD compositions, and in particular, chlorine dioxide-containing oxy-chlorine complexes, is not completely understood. The manufacture or production of certain chlorine dioxide precursors is described by Gordon, U.S. Patent 3,585,147 and Lovely, U.S. Patent 3,591,515. Specific examples of commercially available and useful stabilized chlorine dioxide include, for example, ANTHIUM DIOXCIDE and FERMASURE, available from E. I. du Pont de Nemours and Company, Wilmington DE; OXINE and PUROGENE, available from Bio-Cide International, Inc., Norman, OK.

SCD may be provided as a solution of the one or more chlorine dioxide-containing oxy-chlorine complexes, one or more chlorite-containing compounds, or one or more other entities capable of forming chlorine dioxide when exposed to acid, and combinations thereof. The solution provides SCD in a liquid medium at a predetermined concentration of "available chlorine dioxide". The concentration of "available chlorine dioxide" is based on complete (100%) release of chlorine dioxide from the one or more chlorine dioxide-containing oxy-chlorine complexes and/or complete (100%) conversion of the one or more chlorite-containing compounds to chlorine dioxide and/or complete (100%) formation of chlorine dioxide from the one or more other entities capable of forming chlorine dioxide. Preferably, the SCD solution has sufficient SCD to have an available chlorine dioxide concentration in the range of about 0.002% to about 40% by weight, preferably, in the range of about 2% to about 25% by weight, more preferably in the range of about 5% to about 15% by weight, based on the total weight of the SCD solution.

SCD may be provided as a solid material, such as a composition comprising an alkali or alkaline earth metal chlorite solid, inert ingredients, and optionally dry activator such as a dry acid.

SCD may also be provided as a mixture (or slurry) comprising a saturated solution of alkali or alkaline earth metal chlorite and additional solid alkali or alkaline earth metal chlorite. Such slurries provide a liquid SCD with a higher active ingredient level than available in solution form.

The invention is hereinafter described in terms of SCD as stabilized alkali metal chlorite, more specifically sodium chlorite ($NaClO_2$). Typically sodium chlorite is used as an aqueous solution comprising 5 - 22% by weight, based on weight of the solution. Hereinafter SCD concentrations are described in terms of the concentration of chlorine dioxide available when the chlorite is stoichiometrically converted to chlorine dioxide, "available $ClO_2$". The content of potential chlorine dioxide in 1 g of sodium chlorite is 0.597 g. Sodium chlorite solutions comprising 5 - 22% by weight of sodium chlorite thus contain 2.98 - 13.13% available chlorine dioxide. The generation of $ClO_2$ is illustrated by the following equation (2):

$$5 \ NaClO_2 + 4H^+ \rightarrow 4 \ ClO_2 \ (g) + 2 \ H_2O + Cl\text{-} + 5 \ Na^+ \qquad (2)$$

wherein one mole of $NaClO_2$ provide 0.8 mole of $ClO_2$ (5:4 ratio). (See for example, Ulllmann's Encyclopedia of Industrial Chemistry, Wiley Online Library, http://onlinelibrary.wiley.com/doi/10.1002/14356007.a06_483.pub2/pdf, accessed September 30, 2010.)

Equations providing stoichiometry for generation of $ClO_2$ from other chlorite-containing compounds or from one or more chlorine dioxide-containing oxy-chlorine complexes, or from one or more other entities capable of forming chlorine dioxide when exposed to acid are known to those skilled in the art.

Method for Growth Control

The method of this invention for controlling growth of one or more contaminant microorganisms comprises, consists essentially of or consists of adding a combination of the components (a) stabilized chlorine dioxide (SCD) and (b) peroxide compound (PC), to one or more steps of a fermentation process.

The components SCD and PC may be added separately to the fermentation process. When added separately, the SCD and PC may be added simultaneously (at the same time) or sequentially (at different times) in any order to one or more different process steps.

The components SCD and PC may be added separately or in combination to any of the reactants such as the inoculant, fermentable sugar or process water prior to introducing the reactant and component into the fermentation vessel.

Alternatively, the method may comprise contacting components SCD and PC prior to adding to the fermentation process. Preferably components SCD and PC are contacted prior to adding and are thus added as a single composition to the fermentation process.

The fermentation process may be batch or continuous. In a batch fermentation, preferably the components SCD and PC are added as a single composition (contacted prior to adding) or separately to the fermentation vessel prior to adding a fermentable sugar, more preferably prior to adding any of the reactants. In a continuous fermentation process, components SCD and PC may be added as a single composition in a yeast propagation step or in the contacting step to the fermentation vessel. If more than one fermentation vessel is used in series, preferably the components are added to the first or primary fermentation vessel in the series.

Preferably SCD and PC are added to the process prior to the production of significant amount of ethanol, or prior to the production of significant amounts of organic acid associated with growth of contaminant bacteria. By "significant amounts of ethanol", it is meant that the amount of ethanol in the fermentation broth is not more than 10 % by weight, based on the total volume of the fermentation broth. By "significant amounts of organic acids", it is meant that the amount of organic acids in the fermentation broth is not more than 0.3% by weight, based on the total volume of the fermentation broth. Preferably SCD and PC are added early in the fermentation process, such as during the propagation of inoculant yeast, before, or just after inoculant yeast has been introduced into the fermentation vessel.

In another embodiment, SCD and PC are added to the fermentation process prior to the introduction of inoculant into the fermentation vessel. In this embodiment, SCD and PC may be added to the fermentable sugar or process water or both, prior to introducing either of these reactants to the fermentation vessel and the combined SCD/PC/fermentable sugar and/or SCD/PC/process water is introduced to the fermentation vessel prior to introducing the inoculant to the fermentation vessel. For example SCD and PC may be added to one or more steps of providing the fermentable sugar, such as to one or more steps of a wet mill or dry grind process.

When the fermentation process includes a sugarcane- or sugar beet- or molasses-based fermentable sugar, it is typical to have fermentation vessels in series, having a first, second or even third or more fermentation vessel as a series of vessels where the fermentation broth is successively transferred into each fermentation vessel as part of the fermentation cycle. In this embodiment, SCD and PC are preferably added to the first fermentation vessel.

Alternatively, in a fermentation process where sugarcane or sugar beet or molasses is used as the fermentable sugar, SCD and PC are added to the fermentable sugar prior to contacting the fermentable sugar with inoculant. SCD and PC may be added during the storage of the fermentable sugar. Such step of adding SCD and PC during storage is contemplated herein as a step of the process for providing the fermentable sugar, prior to introducing the fermentable sugar into the fermentation vessel.

SCD and PC may also be added to an empty fermentation vessel, prior to adding the reactants.

In another alternative, for a batch process in which sugarcane or molasses is the fermentable sugar, and in which the inoculant is recycled at the completion of each fermentation cycle, the SCD and PC may be added to a yeast treatment stage (yeast propagation) prior to the beginning of a subsequent batch in a fermentation process. That is, the SCD and PC are added separately, simultaneously, or as a combined composition, into the yeast recycle stream, which is commonly referred to as yeast cream.

In one embodiment, SCD and PC are added to the process water. Such a step of adding SCD and PC to process water is contemplated herein as a step of the process for providing the process water, prior to introducing process water into the fermentation vessel. Process water includes any water that is introduced into the fermentation process from either external sources or recycled from other parts of the fermentation process.

In a molasses-based fermentation process, process water includes water used to dilute incoming molasses prior to fermentation, water added as part of the yeast preparation process, or water recycled from preceding fermentation steps, including yeast recycle stream.

In a dry grind fermentation process, process water includes water added into the steep tank, slurry tank, yeast propagation, or fermentation vessels. The process water may also be a recycle stream in a dry grind batch fermentation process, wherein the recycle stream is a stream produced after fermentation is complete, and is commonly referred to as backset or process condensate. These stages of the dry grind fermentation process are well known to those skilled in the art. See, for example, R. J. Bothast and M. A. Schlicher, Applied Microbiology and Biotechnology as cited hereinabove.

The fermentation process may further comprise or alternatively comprise following the contacting step, adding SCD and PC to the product of the contacting step which product comprises ethanol, separating ethanol from the product. Alternatively, the fermentation process may further comprise, following the contacting step, separating the ethanol from the remaining product, wherein the remaining product is fed, for example, into a beer-well, or a thin stillage tank and adding the SCD and PC to the beer-well or thin stillage tank.

Stabilized chlorine dioxide (SCD) and the peroxide compound (PC) are added to the fermentation process in an amount to provide an effective amount to control growth of one or more contaminant microorganisms. Typically SCD is added in an amount to provide a concentration of the stabilized chlorine dioxide of 0.0002% to 5% by weight, based on the total volume of the fermentation broth. Typically the peroxide compound (PC) is also added to the fermentation process in an amount to provide a concentration of 0.0002% to 5% by weight, based on the total volume of the fermentation broth. Preferably, SCD and PC are added to the fermentation process to provide a concentration of SCD of 0.0005% to 1%, more preferably from 0.002 to 1 % by weight, and a concentration of PC of 0.001 to 1%, more preferably from 0.002 to 1% by weight, based on the total volume of the fermentation broth. Most preferably, SCD and PC are added to result in a concentration of SCD in the fermentation broth of 0.005 to 0.5% by weight, and a concentration of PC of 0.005 to 0.5% by weight, based on the total volume of the fermentation broth.

Preservation method

As an embodiment of the present invention for controlling growth of contaminant microorganisms in a fermentation process, SCD and PC may be added as a step in providing the fermentable sugar. More particularly, as defined herein, the fermentation process comprises process steps to produce and store carbohydrate feedstock wherein the carbohydrate feedstock comprises a fermentable sugar or is used to prepare a fermentable sugar. Thus, the step of providing a fermentable sugar may comprise contacting a carbohydrate feedstock with SCD and PC. Thus, the method of this invention is also useful to prevent degradation in storage of a carbohydrate feedstock.

The carbohydrate content of the feedstock is at least 1% and preferably 1 to 70%, by weight, based on the total feedstock weight. The stabilized chlorine dioxide and peroxide compound are added in effective amounts. Typically the stabilized chlorine dioxide is added in an amount of 10 ppm to 1000 ppm of the stabilized chlorine dioxide, based on total weight of the feedstock, and the peroxide compound is typically added in an amount of 10 ppm to 1000 ppm of the peroxide compound, based on the total weight of the feedstock, wherein all amounts herein expressed as ppm are on a weight/weight basis. Preferably, stabilized chlorine dioxide is added in an amount of 50 ppm to 500 ppm, more preferably 100 ppm to 500 ppm, and most preferably 100 ppm to 200 ppm of stabilized chlorine dioxide, based on the total weight of the feedstock. Preferably, the peroxide compound is added in an amount of 50 ppm to 500 ppm, more preferably 100 ppm to 500 ppm, and most preferably 100 ppm to 200 ppm of the peroxide compound, based on total weight of the feedstock.

In this method, SCD and PC are contacted with a carbohydrate feedstock in an effective amount to protect the carbohydrate from the growth of undesirable microorganisms and thus to prevent deterioration of the feedstock. Deterioration of the feedstock can be determined by the populations of contaminant microorganisms present, or the concentration of microbial metabolites, such as organic acids, that generally indicate unintended and undesirable microbial activity in the feedstock. Microorganisms are thus substantially prevented from proliferating in the stored or transported feedstock following the addition of SCD and PC.

The present invention may be used to control contaminant microorganisms during storage and transport, preserving the carbohydrate feedstock.

Surprisingly, the carbohydrate feedstock treated according to this invention remains stable for at least one month. By "stable", it is meant herein the addition of SCD and PC preserves the carbohydrate feedstock, where "preserve" is defined hereinabove as preventing reaction of or consumption of carbohydrate by contaminant microorganisms. A stable carbohydrate feedstock does not undergo an increase in the microbial population in the feedstock of more than 1 $\log_{10}$ CFU/ml or 1 $\log_{10}$ CFU/g. CFU, an abbreviation for colony forming unit, is a measure of microbial population in the feedstock. CFU is used to determine the number of viable microbial cells in a sample per unit volume or per unit mass, or the degree of microbial contamination in samples. A second measure of change is pH of the preserved feedstock. The pH of a properly preserved feedstock should not change by more than 0.5 pH units. However, as previously stated, pH change may not be sufficient under all circumstances to monitor preservation of a carbohydrate feedstock.

The carbohydrate feedstock is defined hereinabove.

In the present invention, the combination of SCD and PC is used as a preservative for carbohydrate feedstocks to impede contaminant microorganism activity and subsequent deterioration of the carbohydrate feedstock as a step in providing a fermentable sugar. Contaminant microorganisms include bacteria as disclosed in WO 2007/149450 and contaminant yeast as disclosed in U.S. Patent Application Serial No. 12/467,728, filed May 18, 2009 (US 2010/0291649) . The combination inhibits growth of certain bacteria that cause undesired decomposition of carbohydrates such as simple sugars to deleterious acids and also selectively to reduce the activity of contaminant yeasts.

## EXAMPLES

Example 1

In this example, stabilized chlorine dioxide (SCD) and hydrogen peroxide (HP) were used in a checkerboard assay to determine whether their interaction was synergistic against *Lactobacillus brevis,* a common contaminant in fermentation processes. The checkerboard assay method is used to analyze interactions between compounds by mixing them in increasing concentrations to determine whether the compounds act synergistically, additively or antagonistically. (See, M. Najjar, D. Kashtanov, M. Chikindas, Letters in Applied Microbiology, vol. 45 (2007) 13-18.) L. *brevis* was cultured overnight in MRS broth (deMan Rogosa and Sharp broth, Difco Laboratories, Inc., Sparks, MD) at 32°C. The culture was then diluted and resuspended in fresh MRS broth adjusted to pH 4.8. and then added (150 μl) to the wells of a 96-well plate (Becton, Dickinson and Co., Franklin Lakes, NJ). SCD (FermaSure® fermentation additive, E. I. du Pont de Nemours and Co., Wilmington DE) and hydrogen peroxide (30%, VWR International LLC, West Chester, PA) were then added at the concentrations shown in the "checkerboard" plate diagram below. The plate was covered and incubated for 28 hours at 32°C. Following incubation, the plate was removed and the optical density of each well was determined using a plate reader at 630 nm. Based on the optical density, each well was scored to determine whether there was no inhibition (NI), partial inhibition (PI), or complete inhibition (CI).

Table 1. Checkerboard assay to determine response of *Lactobacillus brevis* to treatment with SCD and hydrogen peroxide in MRS broth at pH 4.8.

| | | SCD | | | | | | |
|---|---|---|---|---|---|---|---|---|
| | | 0 ppm | 5 ppm | 12.5 ppm | 15 ppm | 25 ppm | 37.5 ppm | 50 ppm |
| HP | 0 ppm | NI | NI | PI | PI | PI | PI | CI |
| | 10 ppm | PI | PI | PI | PI | PI | PI | |
| | 25 ppm | PI | PI | PI | PI | PI | CI | |
| | 50 ppm | PI | PI | PI | PI | CI | | |
| | 100 ppm | CI | CI | CI | CI | | | |

To determine whether any synergistic activity occurred between SCD and hydrogen peroxide, the fractional inhibitory concentration (FIC) was determined as above using the formula:

$$FIC= [SCD]/MIC_{SCD} + [HP]/MIC_{HP}.$$

where

[SCD] = SCD concentration that gives partial inhibition (PI)
[HP] = HP concentration that gives partial inhibition (PI)
$MIC_{SCD}$ = concentration of SCD that gives complete inhibition
$MIC_{HP}$ = concentration of HP that gives complete inhibition

For the checkerboard assay above, the FIC is determined to be 0.35, indicating a synergistic effect for the interaction between SCD and hydrogen peroxide against *Lactobacillus brevis.* Thus, Example 1 demonstrates that the combination of hydrogen peroxide and SCD will enable a reduction in the dose of SCD required to inactivate bacteria in a fermentation process.

Example 2

In this example, corn mash was obtained from a commercial ethanol plant immediately after the liquefaction step. The mash was cooled to room temperature and then inoculated with *L. brevis* cultures prepared as was described in Example 1. 50 ml portions of the inoculated corn mash were treated with SCD, HP and a combination of both as follows:

| Sample | Treatment |
|--------|-----------|
| 1 | Untreated |
| 2 | 50 ppm HP |
| 3 | 100 ppm HP |
| 4 | 50 ppm HP + 500 ppm SCD |
| 5 | 500 ppm SCD |
| 6 | 1000 ppm SCD |

Following treatment, the samples were incubated for 2 hours in a shaking incubator. Surviving bacteria in each sample was enumerated by plating serial dilutions of each on MRS agar plates (available from Difco Laboratories, Inc., Sparks, MD) and further incubating the plates for 48 hours at 32°C.

Table 2: Reduction of lactic acid bacteria in corn mash treated with HP, SCD and a combination of HP and SCD

| Treatment | Viable cells after 2 hours (Log CFU/g) |
|-----------|----------------------------------------|
| Untreated | 7.50 |
| 50 ppm HP | 7.48 |
| 100 ppm HP | 5.97 |
| 50 ppm HP + 500 ppm SCD | 3.52 |
| 500 ppm SCD | 5.38 |
| 1000 ppm SCD | 5.28 |

The number of surviving bacteria in each sample is shown in Table 2. The highest reduction in the number of viable bacteria was obtained with the combination of 50 ppm HP and 500 ppm SCD. Individual treatments with 100 ppm HP, and 1000 ppm SCD, representing twice the concentration of each in the combined treatment, was not as effective as the combined treatment. Thus, Example 2 demonstrates that the combination of HP and SCD was more effective in reducing the number of contaminant bacteria in a fermentation process than either of the compounds by themselves.

**Claims**

1. A method for controlling growth of contaminant microorganisms in a fermentation process comprising adding the components (a) stabilized chlorine dioxide (SCD) and (b) a peroxide compound, (PC) to one or more steps of a fermentation process, wherein the fermentation process comprises (i) providing a fermentation vessel; (ii) providing a yeast inoculant, a fermentable sugar and process water; (iii) introducing separately, or in any combination, the inoculant, fermentable sugar and process water into a fermentation vessel to provide a fermentation broth; and (iv) contacting the inoculant with the fermentable sugar in the fermentation vessel at a temperature at which the inoculant converts the fermentable sugar to ethanol.

2. The method of claim 1 further comprising adding nutrients to one or more of the inoculant, fermentable sugar and process water or directly to the fermentation vessel.

3. The method of claim 2 wherein the stabilized chlorine dioxide and peroxide compound are added separately to one or more steps of the fermentation process.

4. The method of claim 2 further comprising contacting the stabilized chlorine dioxide and peroxide compound prior to adding the stabilized chlorine dioxide and peroxide compound to one or more steps of the fermentation process.

5. The method of claim 2 wherein stabilized chlorine dioxide or peroxide compound is added to the inoculant, fermentable sugar or process water prior to introducing the inoculant, fermentable sugar or process water into the fermentation vessel.

6. The method of claim 1 wherein the yeast inoculant is a yeast inoculum produced by contacting a yeast starter culture and a nutrient composition in a propagation tank.

7. The method of claim 1 or 5 wherein the fermentation process is a batch fermentation, and the stabilized chlorine dioxide and peroxide compound are added to the fermentation vessel prior to adding the fermentable sugar to the fermentation vessel.

8. The method of claim 5 wherein the fermentation process is a continuous fermentation process and stabilized chlorine dioxide and peroxide compound are combined and added as a single composition to the yeast propagation step to produce the yeast inoculum or during the contacting step (iv).

9. The method of claim 2 wherein the fermentable sugar is derived from corn using either a dry grind process or a wet mill process, or wherein the fermentable sugar is sugarcane- or sugar beet- or molasses-based fermentable sugar.

10. The method of claim 2 wherein the stabilized chlorine dioxide is a chlorine dioxide-containing oxy-chlorine complex selected from the group consisting of a complex of chlorine dioxide with carbonate, a complex of chlorine dioxide with bicarbonate and mixtures thereof.

11. The method of claim 2 wherein the stabilized chlorine dioxide is a metal chlorite, preferably wherein the metal chlorite is sodium chlorite.

12. The method of claim 2 wherein the peroxide compound is selected from the group consisting of a hydroperoxide, organic peroxide, inorganic peroxide or peroxygen-releasing compound, preferably wherein the peroxide compound is a hydroperoxide, or hydrogen peroxide, and preferably wherein the metal chlorite is sodium chlorite.

13. The method of claim 1 wherein stabilized chlorine dioxide is added in an amount to provide a concentration of the stabilized chlorine dioxide of 0.0002% to 5%, based on the total volume of the fermentation broth; and the peroxide compound is added in an amount to provide a concentration of 0.0002% to 5% based on the total volume of the fermentation broth.

14. The method of claim 1 further comprising following the contacting step, adding stabilized chlorine dioxide and peroxide compound to the product of the contacting step.

15. The method of claim 1 wherein the step of providing the fermentable sugar comprises producing and storing a carbohydrate feedstock wherein stabilized chlorine dioxide and peroxide compound are added to the carbohydrate feedstock and wherein the carbohydrate feedstock comprises a fermentable sugar and further wherein the carbohydrate content of the feedstock is at least 1% by weight, based on the total feedstock weight and the stabilized chlorine dioxide is added in an amount of 10 ppm to 1000 ppm of stabilized chlorine dioxide, based on total weight of the feedstock, and the peroxide compound is added in an amount of 10 ppm to 1000 ppm of peroxide compound, based on the total weight of the feedstock, preferably wherein the feedstock is either a sugar-based feedstock or a cellulose feedstock.

16. The method of claim 15 wherein stabilized chlorine dioxide is added in an amount of 50 ppm to 500 ppm, based on the total weight of the feedstock and the peroxide compound is added in an amount of 50 ppm to 500 ppm, based on total weight of the feedstock and wherein the carbohydrate content of the feedstock is 1 to 70%, by weight, based on the total feedstock weight.

17. A method according to claim 1 wherein the fermentation process further comprises after step (iv), either adding SCD and PC to the product of the contacting step which product comprises ethanol, or separating the ethanol from the product of the contacting step which product comprises ethanol to provide a remaining product, wherein the remaining product is fed, into a beer-well or a thin stillage tank and adding the SCD and PC to the beer-well or thin stillage tank.

**Patentansprüche**

1. Verfahren zum Bekämpfen des Wachstums von verunreinigenden Mikroorganismen bei einem Fermentierungsvorgang, umfassend das Zugeben der Komponenten (a) stabilisiertes Chlordioxid (SCD) und (b) eine Peroxidverbindung (PV) einem oder mehreren Schritten eines Fermentierungsvorgangs, wobei der Fermentierungsvorgang Folgendes umfasst: (i) das Bereitstellen eines Fermentierungsgefäßes; (ii) das Bereitstellen eines Hefeimpfmittels, eines fermentierbaren Zuckers und von Prozesswasser); (iii) das Einführen, getrennt oder in Kombination, des Impfmittels, fermentierbaren Zuckers und Prozesswassers in ein Fermentierungsgefäß, um eine Fermentierungsbouillon bereitzustellen; und (iv) das Kontaktieren des Impfmittels mit dem fermentierbaren Zucker in dem Fermentierungsgefäß bei einer Temperatur, bei der das Impfmittel den fermentierbaren Zucker in Ethanol umwandelt.

2. Verfahren nach Anspruch 1, ferner das Zugeben von Nährstoffen zu einem oder mehreren von dem Impfmittel, fermentierbaren Zucker und Prozesswasser oder direkt in das Fermentierungsgefäß umfassend.

3. Verfahren nach Anspruch 2, wobei das stabilisierte Chlordioxid und die Peroxidverbindung getrennt einem oder mehreren Schritten des Fermentierungsvorgangs zugegeben werden.

4. Verfahren nach Anspruch 2, ferner das Kontaktieren des stabilisierten Chlordioxids und der Peroxidverbindung vor Zugeben des stabilisierten Chlordioxids und der Peroxidverbindung einem oder mehreren Schritten des Fermentierungsvorgangs umfassend.

5. Verfahren nach Anspruch 2, wobei das stabilisierte Chlordioxid oder die Peroxidverbindung dem Impfmittel, fermentierbaren Zucker oder Prozesswasser vor Einführen des Impfmittels, fermentierbaren Zuckers oder Prozesswassers in das Fermentierungsgefäß zugegeben werden.

6. Verfahren nach Anspruch 1, wobei das Hefeimpfmittel ein Hefeinokulum ist, das durch Kontaktieren einer Hefestarterkultur und einer Nährstoffzusammensetzung in einem Fortpflanzungstank hergestellt wird.

7. Verfahren nach Anspruch 1 oder 5, wobei der Fermentierungsvorgang eine chargenweise Fermentierung ist und das stabilisierte Chlordioxid und die Peroxidverbindung in das Fermentierungsgefäß vor Eingeben des fermentierbaren Zuckers in das Fermentierungsgefäß eingegeben werden.

8. Verfahren nach Anspruch 5, wobei der Fermentierungsvorgang ein kontinuierlicher Fermentierungsvorgang ist und das stabilisierte Chlordioxid und die Peroxidverbindung kombiniert werden und als einzige Zusammensetzung zu dem Hefevermehrungsschritt, um das Hefeinokulum herzustellen, oder während des Kontaktierschritts (iv) zugegeben werden.

9. Verfahren nach Anspruch 2, wobei der fermentierbare Zucker von Mais unter Anwendung eines Trockenmahlverfahrens oder eines Nassmahlverfahrens abgeleitet wird oder wobei der fermentierbare Zucker fermentierbarer Zucker auf der Basis von Zuckerrohr oder Zuckerrübe oder Melasse ist.

10. Verfahren nach Anspruch 2, wobei das stabilisierte Chlordioxid ein Chlordioxid enthaltender Oxychlorkomplex ist ausgewählt aus der Gruppe bestehend aus einem Komplex von Chlordioxid mit Carbonat, einem Komplex von Chlordioxid mit Bicarbonat und Mischungen davon.

11. Verfahren nach Anspruch 2, wobei das stabilisierte Chlordioxid ein Metallchlorit ist, wobei bevorzugt das Metallchlorit Natriumchlorit ist.

12. Verfahren nach Anspruch 2, wobei die Peroxidverbindung aus der Gruppe ausgewählt ist bestehend aus Hydroperoxid, organischem Peroxid, anorganischem Peroxid oder Peroxygen freigebender Verbindung, wobei bevorzugt die Peroxidverbindung ein Hydroperoxid oder Wasserstoffperoxid ist und wobei bevorzugt das Metallchlorit Natriumchlorit ist.

13. Verfahren nach Anspruch 1, wobei das stabilisierte Chlordioxid in einer Menge zugegeben wird, um eine Konzentration des stabilisierten Chlordioxids von 0,0002 % bis 5 %, auf das Gesamtvolumen der Fermentierungsbouillon bezogen, bereitzustellen; und die Peroxidverbindung in einer Menge zugegeben wird, um eine Konzentration von 0,0002 % bis 5 %, auf das Gesamtvolumen der Fermentierungsbouillon bezogen, bereitzustellen.

**14.** Verfahren nach Anspruch 1, ferner auf den Kontaktierschritt hin das Zugeben von stabilisiertem Chlordioxid und Peroxidverbindung zu dem Produkt des Kontaktierschritts umfassend.

**15.** Verfahren nach Anspruch 1, wobei der Schritt des Bereitstellens des fermentierbaren Zuckers das Herstellen und Lagern eines Kohlehydratausgangsmaterials umfasst, wobei stabilisiertes Chlordioxid und Peroxidverbindung dem Kohlehydratausgangsmaterial zugegeben werden und wobei das Kohlehydratausgangsmaterial einen fermentierbaren Zucker umfasst und wobei ferner der Kohlehydratgehalt des Ausgangsmaterials mindestens 1 Gew.-%, auf das gesamte Ausgangsmaterialgewicht bezogen, beträgt und das stabilisierte Chlordioxid in einer Menge von 10 ppm bis 1000 ppm stabilisiertes Chlordioxid, auf das Gesamtgewicht des Ausgangsmaterials bezogen, zugegeben wird und die Peroxidverbindung in einer Menge von 10 ppm bis 1000 ppm Peroxidverbindung, auf das Gesamtgewicht des Ausgangsmaterials bezogen, zugegeben wird, wobei bevorzugt das Ausgangsmaterial entweder ein Ausgangsmaterial auf der Basis von Zucker oder ein Celluloseausgangsmaterial ist.

**16.** Verfahren nach Anspruch 15, wobei das stabilisierte Chlordioxid in einer Menge von 50 ppm bis 500 ppm, auf das Gesamtgewicht des Ausgangsmaterials bezogen, zugegeben wird und die Peroxidverbindung in einer Menge von 50 ppm bis 500 ppm, auf das Gesamtgewicht des Ausgangsmaterials bezogen, zugeben wird und wobei der Kohlehydratgehalt des Ausgangsmaterials 1 bis 70 Gew.-%, auf das gesamte Ausgangsmaterialgewicht bezogen, beträgt.

**17.** Verfahren nach Anspruch 1, wobei der Fermentierungsvorgang ferner nach Schritt (iv) entweder das Zugeben von SCD und PV zu dem Produkt des Kontaktierschritts, welches Produkt Ethanol umfasst, oder das Trennen des Ethanols von dem Produkt des Kontaktierschritts umfasst, welches Produkt Ethanol umfasst, um ein verbleibendes Produkt bereitzustellen, wobei das verbleibende Produkt in einen Bierbrunnen oder einen Dünnschlempetank eingegeben wird, und das Zugeben von SCD und PV zu dem Bierbrunnen oder Dünnschlempetank.

## Revendications

**1.** Procédé de régulation de la croissance de micro-organismes contaminants dans un procédé de fermentation comprenant l'addition des composants (a) dioxyde de chlore stabilisé (SCD) et (b) composé peroxyde (PC), à une ou plusieurs étapes d'un procédé de fermentation, dans lequel le procédé de fermentation comprend (i) la fourniture d'une cuve de fermentation; (ii) la préparation d'un inoculant type levure, d'un sucre fermentescible et d'eau de procédé; (iii) l'introduction séparément, ou en n'importe quelle combinaison, de l'inoculant, du sucre fermentescible et de l'eau de procédé dans une cuve de fermentation pour fournir un bouillon de fermentation; et (iv) la mise en contact de l'inoculant avec le sucre fermentescible dans la cuve de fermentation à une température à laquelle l'inoculant convertit le sucre fermentescible en éthanol.

**2.** Procédé selon la revendication 1 comprenant en outre l'addition de nutriments à l'un ou plusieurs de l'inoculant, du sucre fermentescible et de l'eau de procédé ou directement à la cuve de fermentation.

**3.** Procédé selon la revendication 2 dans lequel le dioxyde de chlore stabilisé et le composé peroxyde sont ajoutés séparément à une ou plusieurs étapes du procédé de fermentation.

**4.** Procédé selon la revendication 2 comprenant en outre la mise en contact du dioxyde de chlore stabilisé et du composé peroxyde avant l'addition du dioxyde de chlore stabilisé et du composé peroxyde à une ou plusieurs étapes du procédé de fermentation.

**5.** Procédé selon la revendication 2 dans lequel le dioxyde de chlore stabilisé ou le composé peroxyde est ajouté à l'inoculant, au sucre fermentescible ou à l'eau de procédé avant l'introduction de l'inoculant, du sucre fermentescible ou de l'eau de procédé dans la cuve de fermentation.

**6.** Procédé selon la revendication 1 dans lequel l'inoculant type levure est un inoculum levure produit par mise en contact d'un levain à base de levure et d'une composition de nutriment dans une cuve de multiplication.

**7.** Procédé selon la revendication 1 ou 5 dans lequel le procédé de fermentation est une fermentation par lot, et le dioxyde de chlore stabilisé et le composé peroxyde sont ajoutés à la cuve de fermentation avant l'addition du sucre fermentescible à la cuve de fermentation.

**8.** Procédé selon la revendication 5 dans lequel le procédé de fermentation est un procédé de fermentation continu et du dioxyde de chlore stabilisé et un composé peroxyde sont combinés et ajoutés sous la forme d'une composition unique à l'étape de multiplication de la levure pour produire l'inoculum levure ou durant l'étape de mise en contact (iv).

**9.** Procédé selon la revendication 2 dans lequel le sucre fermentescible est dérivé de maïs en utilisant soit un procédé de broyage à sec soit un procédé de broyage à l'état humide, ou dans lequel le sucre fermentescible est du sucre fermentescible à base de canne à sucre ou de betterave sucrière ou de molasses.

**10.** Procédé selon la revendication 2 dans lequel le dioxyde de chlore stabilisé est un complexe oxo-chlore contenant du dioxyde de chlore sélectionné dans le groupe constitué d'un complexe de dioxyde de chlore avec un carbonate, un complexe de dioxyde de chlore avec du bicarbonate et leurs mélanges.

**11.** Procédé selon la revendication 2 dans lequel le dioxyde de chlore stabilisé est un chlorite métallique, préférablement dans lequel le chlorite métallique est le chlorite de sodium.

**12.** Procédé selon la revendication 2 dans lequel le composé peroxyde est sélectionné dans le groupe constitué d'un hydroperoxyde, d'un peroxyde organique, d'un peroxyde inorganique ou d'un composé de libération de peroxygène, préférablement dans lequel le composé peroxyde est un hydroperoxyde, ou un peroxyde d'hydrogène, et préférablement dans lequel le chlorite métallique est le chlorite de sodium.

**13.** Procédé selon la revendication 1 dans lequel le dioxyde de chlore stabilisé est ajouté en une quantité permettant de fournir une concentration du dioxyde de chlore stabilisé de 0,0002 % à 5 %, sur la base du volume total du bouillon de fermentation; et le composé peroxyde est ajouté en une quantité permettant de fournir une concentration de 0,0002 % à 5 % sur la base du volume total du bouillon de fermentation.

**14.** Procédé selon la revendication 1 comprenant en outre suite à l'étape de mise en contact, l'addition de dioxyde de chlore stabilisé et d'un composé peroxyde au produit de l'étape de mise en contact.

**15.** Procédé selon la revendication 1 dans lequel l'étape de fourniture du sucre fermentescible comprend la production et le stockage d'une charge d'alimentation hydrate de carbone où le dioxyde de chlore stabilisé et le composé peroxyde sont ajoutés à la charge d'alimentation hydrate de carbone et dans lequel la charge d'alimentation hydrate de carbone comprend un sucre fermentescible et en outre dans lequel la teneur en hydrate de carbone de la charge d'alimentation est d'au moins 1 % en poids, sur la base du poids total de la charge d'alimentation et le dioxyde de chlore stabilisé est ajouté en une quantité de 10 ppm à 1 000 ppm de dioxyde de chlore stabilisé, sur la base du poids total de la charge d'alimentation, et le composé peroxyde est ajouté en une quantité de 10 ppm à 1 000 ppm de composé peroxyde, sur la base du poids total de la charge d'alimentation, préférablement dans lequel la charge d'alimentation est soit une charge d'alimentation à base de sucre soit une charge d'alimentation à base de cellulose.

**16.** Procédé selon la revendication 15 dans lequel le dioxyde de chlore stabilisé est ajouté en une quantité de 50 ppm à 500 ppm, sur la base du poids total de la charge d'alimentation et le composé peroxyde est ajouté en une quantité de 50 ppm à 500 ppm, sur la base du poids total de la charge d'alimentation et dans lequel la teneur en hydrate de carbone de la charge d'alimentation est de 1 à 70%, en poids, sur la base du poids total de la charge d'alimentation.

**17.** Procédé selon la revendication 1 dans lequel le procédé de fermentation comprend en outre après l'étape (iv), soit l'addition de SCD et de PC au produit de l'étape de mise en contact lequel produit comprend de l'éthanol, soit la séparation de l'éthanol du produit de l'étape de mise en contact lequel produit comprend de l'éthanol pour fournir un produit restant, le produit restant étant alimenté, au niveau d'un réservoir de garde ou d'une cuve de résidus solubles de distillation et l'addition du SCD et du PC au réservoir de garde ou à la cuve de résidus solubles de distillation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007149450 A
- US 2010291649 A
- CA 2300807
- US 2002064565 A
- US 3585147 A
- US 3591515 A
- US 46772809 A
- US 20100291649 A

### Non-patent literature cited in the description

- **J. Y. ZHU et al.** *Bioresource Technology,* 2010, vol. 101 (13), 4992-5002
- **A. L. DEMAIN.** *J. Ind. Microbiol. Biotechnol.,* 2009, vol. 36 (3), 319-332
- **H. V. AMORIM et al.** The Alcohol Textbook. Nottingham University Press, 2009, 39-46
- **R. J. BOTHAST ; M. A. SCHLICHER.** *Applied Microbiology and Biotechnology,* 2005, vol. 67 (1), 19-25
- **E. BELLISSIMI et al.** *Process Biochemistry,* 2005, vol. 40 (6), 2205-2213
- **M. KNAUF et al.** *Sugar Industry,* 2006, vol. 131, 753-758
- **E. A. N. FERNANDES et al.** *Journal of Radioanalytical and Nuclear Chemistry,* 1998, vol. 234 (1-2), 113-119
- **L. C. BASSO et al.** *FEMS Yeast Res.,* 2008, 1155-1163
- **JACQUES, K.A. ; LYONS, T.P. ; KELSALL, D.R.** The Alcohol Textbook. Nottingham University Press, 2003, 423-424
- Ulllmann's Encyclopedia of Industrial Chemistry. Wiley Online Library, 30 September 2010
- **M. NAJJAR ; D. KASHTANOV ; M. CHIKINDAS.** *Letters in Applied Microbiology,* 2007, vol. 45, 13-18